# EUROPÄISCHE PATENTSCHRIFT

(11) **EP 0 647 613 B1**
(45) Veröffentlichungstag und Bekanntmachung des Hinweises auf die Patenterteilung: **14.01.1998**
(21) Anmeldenummer: 94111214.6
(22) Anmeldetag: 19.07.1994
(51) Int. Cl.: C07C 231/12, C07C 233/36, C11D 1/90

(54) **Verfahren zur Herstellung hochkonzentrierter fliessfähiger wässriger Lösungen von Betainen**
Process for the preparation of highly concentrated, freely flowable, aqueous solutions of betaines
Procédé pour la préparation de dispersions aqueuses de bétaines fortement concentrées et fluides

(30) Priorität: 08.10.1993 DE 4334467
(43) Veröffentlichungstag der Anmeldung: 12.04.1995
(73) Patentinhaber: Witco Surfactants GmbH, D-36396 Steinau an der Strasse (DE)
(72) Erfinder: Hamann, Ingo, Dr., D-63619 Bad Orb (DE); Köhle, Hans-Jürgen, Dr., D-36381 Schlüchtern (DE); Wehner, Winfried, Dr., D-36119 Neuhof (DE)

(56) Entgegenhaltungen:
- EP-A- 0 353 580
- EP-A- 0 557 835
- DE-C- 3 726 322
- DE-C- 4 207 386
- US-A- 3 225 074

## Beschreibung

Die Erfindung betrifft ein Verfahren zur Herstellung hochkonzentrierter fließfähiger wässriger Lösungen von Betainen, welche Feststoffgehalte von mehr als 40 Gew.-%, vorzugsweise mehr als 50 Gew.-%, enthalten.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, welcher auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-PS 3 225 074). Im allgemeinen werden dabei tertiäre Aminstickstoffatome enthaltende Verbindungen mit ω-Halogencarbonsäuren oder deren Salze in wässrigen oder wasserhaltigen Medien umgesetzt. Als tertiäre Aminstickstoffatome enthaltende Verbindungen werden insbesondere Fettsäureamide der allgemeinen Formel (II)

R³-CONH-(CH₂)ₘ-NR⁴R⁵ (II)

eingesetzt, worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedenen Alkylreste mit 1 - 4 C-Atomen bedeuten und m = 1 - 3 sein kann.

Der Alkylrest R³ kann sich hierbei von den natürlichen oder synthetischen Fettsäuren mit 6 - 20 C-Atomen, vorzugsweise von den natürlichen pflanzlichen oder tierischen Fettsäuren mit 8 - 18 C-Atomen ableiten sowie deren natürlich vorkommenden speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Linolsäure, Linolensäure, Ricinolsäure in Betracht.

Bevorzugt werden die natürlich vorkommenden Fettsäuremischungen mit einer Kettenlänge von 8 - 18 C-Atomen, wie Kokosfettsäure oder Palmkernfettsäure, welche gegebenenfalls durch geeignete Hydrierungsmethoden gehärtet werden können.

Mit diesen Fettsäuren bzw. Fettsäuremischungen wird durch übliche Kondensationsreaktion bei 140 - 200 °C mit Aminen der allgemeinen Formel (III)

H₂N-(CH₂)ₘ-NR⁴R⁵ (III)

- in welcher R⁴ und R⁵ und m die in der Formel (II) genannte Bedeutung haben - zu den Fettsäureamiden mit tertiären Stickstoffatomen der allgemeinen Formel (II) umgesetzt.

Die anschließende Quaternierungsreaktion zu Betainen der Formel (IV)

R³-CONH-(CH₂)ₘ-N⁺R⁴R⁵(CH₂)_{y}COO⁻ (IV)

worin R³, R⁴, R⁵ und die m die gleiche Bedeutung wie in den Formeln (II) und (III) haben und y = 1, 2, 3 sein kann, kann nach den literaturbekannten Verfahren durchgeführt werden.

Dem Fettsäureamid der Formel (II) werden dabei in der Regel in wässrigem Medium ω-Halogenalkylcarbonsäuren, vorzugsweise das Natriumsalz der Chloressigsäure, zugesetzt und bei einer mehrstündigen Reaktion bei ca. 80 - 100 °C die Quaternierung vollzogen. Je nach eingesetzter Fettsäure bzw. Fettsäuremischung muß zum Erhalt der Rührfähigkeit bei fortschreitender Reaktion eine Mindestmenge an Wasser vorhanden sein. Die handelsübliche Konzentration an Betaingehalt der auf diese Weise hergestellten Lösungen liegt daher bei etwa 30 Gew.-% oder darunter.

Zur Einsparung von Lagerungs- und Transportkosten sowie aus formulierungstechnischen Gründen bei der Weiterverarbeitung war aber in vielen Fällen eine höhere Konzentration dringend erwünscht.

In der Vergangenheit sind daher eine Reihe von Verfahren vorgeschlagen worden, welche dieses Problem lösen sollten. So ist aus der DE-PS 3 613 944 ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird.

Abgesehen davon, daß das Verfahren technisch aufwendig und kostenintensiv ist, sind organische Lösungsmittel bei der Weiterverarbeitung in kosmetischen Rezepturen vielfach unerwünscht.

Das Verfahren gemäß DE-PS 3 726 322 kommt zwar ohne organische Lösungsmittel aus, jedoch muß die für die Quaternierungsreaktion erforderliche Wassermenge durch Destillation wieder aus dem Reaktionsprodukt entfernt werden, wobei vor oder nach der Einstellung auf die gewünschte Konzentration der pH-Wert der Lösung durch relativ hohe Säuremengen auf hautuntypische Werte von 1 - 4,5 eingestellt werden muß.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether eingesetzt, welche für eine ausreichende Wasserlöslichkeit 10 - 250 Oxyethyleneinheiten enthalten müssen.

Polyoxyethylenether mit höheren Anteilen an Oxyethyleneinheiten haben sich hinsichtlich ihrer biologischen Abbaubarkeit aber als nicht unproblematisch erwiesen.

Es bestand daher weiterhin ein Bedarf an hochkonzentrierten, fließ- und pumpfähigen, wässrigen Lösungen von Betainen, welche frei von niedrigen Alkoholen wie Methanol, Ethanol, Propanol oder Isopropanol sind.

Überraschenderweise wurde nun gefunden, daß man fließ- und pumpfähige Lösungen von Betainen aus dem Reaktionsgemisch aus Fettsäureamid und ω-Halogenalkylcarbonsäure herstellen kann, insbesondere wenn man dem Fettsäureamid der allgemeinen Formel (II) und dem Natriumsalz der Chloressigsäure vor oder während der Quaternierungsreaktion Verbindungen der allgemeinen Formel (I) 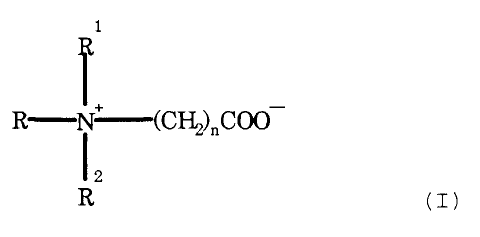 worin R, R¹, R² gleich oder verschieden gegebenenfalls verzweigter, gegebenenenfalls Hydroxylgruppen enthaltende Alkylreste mit 1 - 10 C-Atomen, insbesondere Methylreste und n = 1 - 3, vorzugsweise n = 1, ist, in Mengen von 1 - 5 Gew.-%, vorzugsweise 1 - 4 Gew.-%, bezogen auf die Gesamtmischung, zusetzt.

Ohne weitere Aufkonzentrierungsarbeiten, allein durch entsprechende Bemessung des Wasseranteils bei der Quaternierungsreaktion, werden wässrige Lösungen von Betainen erhalten, welche auch ohne Mitverwendung von kurzkettigen, monofunktionellen Alkoholen ohne weiteres fließ- und pumpfähig sind.

Dies war umso überraschender, als die erfindungsgemäß mitverwendeten Verbindungen der Formel (I) überwiegend fest oder sogar - wie das erfindungsgemäß bevorzugt mitverwendete, in der Zuckerrübe (Beta vulgaris) natürlich vorkommende Trimethylglycin oder "Betain" - kristallin sind.

Gegenstand der Erfindung ist ein Verfahren zur Herstellung hochkonzentrierter fließfähiger wässriger Lösungen von Betainen der allgemeinen Formel (IV)

R³-CONH-(CH₂)ₘ-N⁺R⁴R⁵(CH₂)_{y}COO⁻ (IV)

worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedene Alkylreste mit 1 - 4 C-Atomen bedeuten und m = 1 - 3 und y = 1, 2, 3 sein kann, durch Quaternierung von tertiärem Aminstickstoff enthaltenden Verbindungen mit Halogencarbonsäuren, welches dadurch gekennzeichnet ist, daß der Reaktionsmischung vor oder während der Quaternierungsreaktion mindestens eine der Verbindungen der allgemeinen Formel (I) zugesetzt wird 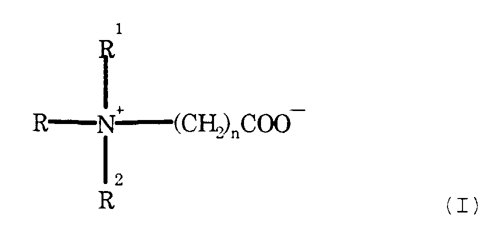 worin R, R¹, R² gleich oder verschieden, gegebenenfalls verzweigt, gegebenenfalls Hydroxylgruppen enthaltende Alkylreste mit 1 - 10 C-Atomen und n = 1 - 3 sein kann.

Weitere erfindungsgmäß mitverwendete Verbindungen der allgemeinen Formel (I) sind Quaternierungsprodukte von Dimethylethanolamin, Methyldiethanolamin oder Alkyl(C₂-C₁₀)Dimethylamin und Monochloressigsäure.

Bereits mit Anteilen von ≤5 Gew.-% an Verbindungen der allgemeinen Formel (I) können fließ- und pumpfähige Mischungen erhalten werden, welche Betaine auf Basis der Fettsäureamide der Formel (II) in Mengen von >45, teilweise >50 Gew.%, bezogen auf Trockensubstanz, enthalten.

Da der Zusatz dieser Verbindungen bei der weiteren Verarbeitung zur Herstellung von Haar- und Körperreinigungsmitteln nicht stört, sie toxikologisch unbedenklich sind und biologisch problemlos abgebaut werden, können, falls im Einzelfalle gewünscht, auch höhere Anteile mitverwendet werden.

Der verfahrensbedingte Anteil an NaCl in der Betainlösung kann in der Regel in der Lösung verbleiben, da zur Viskositätseinstellung von Shampoos oder Duschgelen der Zusatz von Elektrolytsalzen gängige Praxis ist.

Die erfindungsgemäß hergestellten hochkonzentrierten Betainlösungen sind über einen relativ weiten Temperaturbereich stabile homogene und klare Mischungen, welche sich für die weitere Verarbeitung komplikationslos auf die gewünschte Anwendungskonzentration verdünnen und compoundieren lassen.

### Analysenmethoden

### Trockensubstanz:

Die Trockensubstanz wird durch Trocknen des Materials bei 105 °C bis zur Gewichtskonstanz bestimmt. Diese Werte werden bestimmt nach den Einheitsmethoden der Deutschen Gesellschaft für Fettchemie (DGF) : B-II.

### Esterzahl (EZ):

Die Esterzahl ist ein Maß für die in Fetten und technischen Fettsäuren enthaltenen Ester. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): C-V 4.

### Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).

Die Tertiäraminzahl gibt die Anzahl Milligramm Kaliumhydroxid an, die der Tertiäraminbasizität von 1 Gramm der Aminverbindung äquivalent sind (mg KOH/g).

Die Werte werden bestimmt nach American Oil Chemists Society (A.O.C.S.) Official Method Tf 2a-64.

### Natriumchlorid:

Der Gehalt an Natriumchlorid wird potentiometrisch gegen eine Silbernitrat-Maßlösung ermittelt. Als Elektrode wird eine kombinierte Silberchlorid-Elektrode verwendet. Die Werte werden bestimmt nach den Einheitsmethoden der Deutschen Gesellschaft für Fettchemie (DGF): H-III 9.

### Beispiele

### Beispiel 1:

### Herstellung des Aminamids:

98,0 kg Palmkernöl werden in einem Reaktor mit Rührer, Thermometer und Destillationsaufsatz unter Inertgasatmosphäre mit 56,8 kg Dimethylaminopropylamin versetzt und auf 150 - 160 °C erwärmt und unter Rückfluß gekocht. Nach Beendigung der Amidierung (Esterzahl <10 mg KOH/g) wird der Aminüberschuß bei dieser Temperatur im Vakuum abdestilliert. Die Destillation ist vollständig, wenn die Differenz aus Gesamtaminzahl und Tertiäraminzahl geringer als 3 mg KOH/g beträgt. Das erhaltene Aminamid hat eine GAZ von 170,6 mg KOH/g, eine TAZ von 168,6 mg KOH/g und eine Esterzahl von 2,8 mg KOH/g.

### Quaternierung:

12,3 kg Monochloressigsäure-Lösung (80 %) werden unter Kühlung in einem Reaktor mit Rührer, Innenthermometer und pH-Messer mit 37,0 kg Wasser verdünnt und mit 8,1 kg Natriumhydroxid-Lösung (50 %) vorsichtig neutralisiert. Nach der Neutralisation wird die Natriumchloracetat-Mischung mit 3,0 kg Trimethylglycin versetzt und auf 70 - 80 °C erhitzt. Nach Zusatz von 32,5 kg des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 800 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 640 g 50prozentiger Citronensäure-Lösung auf 5,1 ein.

Das Endprodukt ist eine klare, mittelviskose Flüssigkeit mit einem Trockenrückstand von 50,1 % und einem Natriumchlorid-Gehalt von 6,4 %.

### Beispiel 2:

### Herstellung des Aminamids:

95,0 kg gehärtetes Kokosöl werden in einem Reaktor mit Rührer, Thermometer und Destillationsaufsatz unter Inertgasatmosphäre mit 52,8 kg Dimethylaminopropylamin versetzt und auf 150 - 160 °C erwärmt. Nach Beendigung der Amidierung (Esterzahl <10 mg KOH/g) wird der Aminüberschuß bei dieser Temperatur im Vakuum abdestilliert. Die Destillation ist vollständig, wenn die Differenz aus Gesamtaminzahl und Tertiäraminzahl geringer als 3 mg KOH/g beträgt. Das erhaltene Aminamid hat eine GAZ von 172,8 mg KOH/g, eine TAZ von 170,6 mg KOH/g und eine Esterzahl von 3,0 mg KOH/g.

### Quaternierung:

12,1 kg Monochloressigsäure-Lösung (80 %) werden unter Kühlung in einem Reaktor mit Rührer, Innenthermometer und pH-Messer mit 45,0 kg Wasser verdünnt und mit 8,1 kg Natriumhydroxid-Lösung (50 %) neutralisiert. Nach der Neutralisation wird die Natriumchloracetat-Mischung mit 1,5 kg Trimethylglycin versetzt und auf 70 - 80 °C erhitzt. Nach Zusatz von 31,5 kg des Aminamids aus Beispiel 2 wird das Reaktionsgemisch bei 80 - 90 °C gerührt. Dabei wird der pH-Wert der Lösung zwischen 8 und 8,5 gehalten. Hierzu werden insgesamt weitere 1000 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 600 g 50prozentiger Citronensäure-Lösung auf 5,2 ein.

Das Endprodukt ist eine klare, mittelviskose Flüssigkeit mit einem Trockenrückstand von 45,0 % und einem Natriumchlorid-Gehalt von 6,0 %.

## Patentansprüche

1. Verfahren zur Herstellung hochkonzentrierter fließfähiger wässriger Lösungen von Betainen der allgemeinen Formel (IV)
R³-CONH-(CH₂)ₘ-N⁺R⁴R⁵(CH₂)_{y}COO⁻ (IV)
worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedene Alkylreste mit 1 - 4 C-Atomen bedeuten und m = 1 - 3 und y = 1, 2, 3 sein kann, durch Quaternierung von tertiärem Aminstickstoff enthaltenden Verbindungen mit Halogencarbonsäuren, dadurch gekennzeichnet, daß der Reaktionsmischung vor oder während der Quaternierungsreaktion mindestens eine der Verbindungen der allgemeinen Formel (I) zugesetzt wird 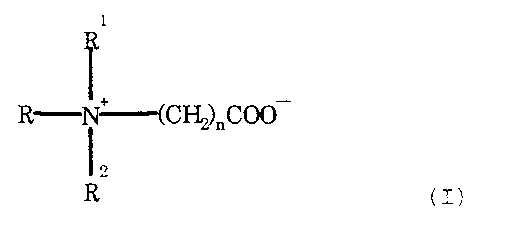 worin R, R¹, R² gleich oder verschieden, gegebenenfalls verzweigt, gegebenenfalls Hydroxylgruppen enthaltende Alkylreste mit 1 - 10 C-Atomen und n = 1 - 3 sein kann.

2. Verfahren gemäß Anspruch 1, dadurch gekennzeichnet, daß R, R¹, R² = CH₃ und n = 1 ist.

3. Verfahren gemäß Anspruch 1, dadurch gekennzeichnet, daß R = CH₃ oder R¹ ist, R¹, R² = -CH₂-CH₂-OH und n = 1 ist.

4. Verfahren gemäß den Ansprüchen 1 und 2, dadurch gekennzeichnet ist, daß die Verbindungen der allgemeinen Formel (I) in Mengen von 1 - 5 Gew.-%, bezogen auf die wässrige Lösung, mitverwendet werden.

5. Verfahren gemäß Anspruch 1, dadurch gekennzeichnet, daß als Betain der allgemeinen Formel (IV) 1-Alkoylamino-3-dimethylamino-propan-3-carboxy-methylbetain mitverwendet wird.

6. Verfahren gemäß Anspruch 5, dadurch gekennzeichnet, daß das Betain auf Basis von Kokosfettsäure zugesetzt wird.

7. Betainlösungen hergestellt gemäß Anspruch 1, dadurch gekennzeichnet, daß sie
1 - 5 Gew.-% Verbindungen der Formel 1
30 - 50 Gew.-% Verbindungen der Formel IV
5 - 8 Gew.-% NaCl
ad 100 Wasser
enthalten.

## Claims

1. Process for the preparation of highly concentrated, free-flowing aqueous solutions of betaines of the general formula (IV)
R³-CONH-(CH₂)ₘ-N⁺R⁴R⁵(CH₂)_{y}COO⁻ (IV)
wherein R³ is the alkyl radical of a fatty acid, which radical is optionally branched, may optionally contain multiple bonds and may optionally contain hydroxyl groups, R⁴ and R⁵ are identical or different alkyl radicals having from 1 to 4 carbon atoms, and m may be from 1 to 3 and y may be 1, 2 or 3, by quaternisation of compounds containing tertiary amine nitrogen with halocarboxylic acids, characterised in that there is added to the reaction mixture before or during the quaternisation reaction at least one of the compounds of the general formula (I) 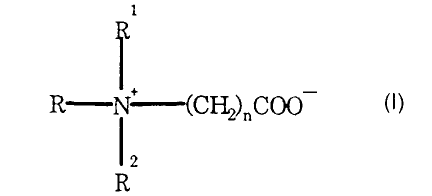 wherein R, R¹ and R² may be identical or different optionally branched alkyl radicals having from 1 to 10 carbon atoms, which radicals optionally contain hydroxyl groups, and n may be from 1 to 3.

2. Process according to claim 1, characterised in that R, R¹ and R² are CH₃ and n is 1.

3. Process according to claim 1, characterised in that R is CH₃ or R¹, R¹ and R² are -CH₂-CH₂-OH, and n is 1.

4. Process according to claims 1 and 2, characterised in that the compounds of the general formula (I) are used concomitantly in amounts of from 1 to 5% by weight, based on the aqueous solution.

5. Process according to claim 1, characterised in that 1-alkanoylamino-3-dimethylamino-propane-3-carboxymethylbetaine is used concomitantly as the betaine of the general formula (IV).

6. Process according to claim 5, characterised in that betaine based on coconut fatty acid is added.

7. Betaine solutions prepared according to claim 1, characterised in that they contain
from 1 to 5% by weight of compounds of formula I
from 30 to 50% by weight of compounds of formula IV
from 5 to 8% by weight of NaCI
ad 100 water.

## Revendications

1. Procédé pour la préparation de solutions aqueuses coulables hautement concentrées de bétaïnes de formule générale (IV) :
R³-CONH-(CH₂)ₘ-N⁺R⁴R⁵(CH₂)_{y}COO⁻ (IV)
dans laquelle R³ représente le reste alkyle d'un acide gras, qui peut être éventuellement ramifié, éventuellement contenir des liaisons multiples, éventuellement contenir des groupes hydroxyles, R⁴ et R⁵, identiques ou différents, peuvent être des restes alkyle avec 1 à 4 atomes de carbone et m = 1 à 3 et y = 1, 2, 3, par quaternisation de composés contenant l'azote amino tertiaire avec des acides halogénocarboxyliques, caractérisé en ce que l'on ajoute au mélange réactionnel, avant ou au cours de la réaction de quaternisation, au moins un des composés de formule générale (I) 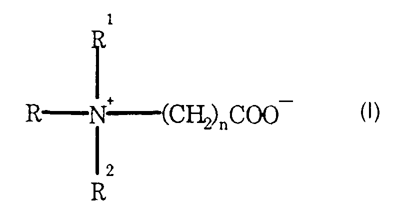 dans laquelle R, R¹, R², identiques ou différents, peuvent être des restes alkyle avec 1 à 10 atomes de carbone, éventuellement ramifiés, éventuellement contenant des groupes hydroxyles, et n = 1 à 3.

2. Procédé selon la revendication 1, caractérisé en ce que R, R¹, R² = CH₃ et n = 1.

3. Procédé selon la revendication 1, caractérisé en ce que R = CH₃ ou R¹, R¹, R² = -CH₂CH₂-OH et n = 1.

4. Procédé selon les revendications 1 et 2, caractérisé en ce que l'on utilise les composés de formule générale (I) à raison de 1 à 5 % en poids par rapport à la solution aqueuse.

5. Procédé selon la revendication 1, caractérisé en ce que l'on utilise en tant que bétaïne de formule générale (IV) la 1-alkylamino-3-diméthylamino-propan-3-carboxyméthylbétaïne.

6. Procédé selon la revendication 5, caractérisé en ce que l'on ajoute la bétaïne à base d'acide gras de coco.

7. Solutions de bétaïnes préparées selon la revendication 1, caractérisées en ce qu'elles contiennent
1 à 5 % en poids de composé de formule I
30 à 50 % en poids de composé de formule IV
5 à 8 % en poids de NaCl
q.s.p. 100 d'eau.
